# EUROPEAN PATENT APPLICATION

(11) **EP 3 666 776 A1**
(43) Date of publication of application: **17.06.2020**
(21) Application number: 18211713.5
(22) Date of filing: 11.12.2018
(51) Int. Cl.: C07D 495/04, A61K 31/519

(54) **HYDRATE OF A GONADOTROPIN-RELEASING HORMONE RECEPTOR ANTAGONIST**

(71) Applicant: Sandoz AG, 4056 Basel (CH)
(72) Inventor: NERDINGER, Sven, 6250 Kundl (AT); STEFINOVIC, Marijan, 6250 Kundl (AT); DOERFER, Melanie, 44227 Dortmund (DE); MUTHUKUMAR, Gomathi Sankar, 44227 Dortmund (DE)
(74) Representative: Kluschanzoff, Harald

(57) **Abstract**

The present invention relates to relugolix *hemi*hydrate and a process for its preparation. The invention also relates to a pharmaceutical composition comprising relugolix *hemi*hydrate, preferably in a predetermined and/or effective amount and at least one pharmaceutically acceptable excipient. The pharmaceutical composition of the present invention can be used as a medicament, in particular for the treatment and/or prophylaxis of a condition selected from the group consisting of endometriosis, uterine fibroids and prostate cancer.

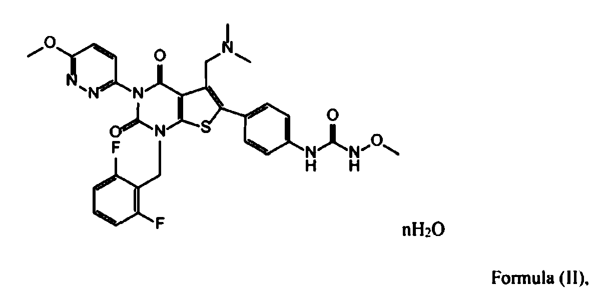

## Description

### FIELD OF THE INVENTION

The present invention relates to relugolix *hemi*hydrate and a process for its preparation. The invention also relates to a pharmaceutical composition comprising relugolix *hemi*hydrate, preferably in a predetermined and/or effective amount and at least one pharmaceutically acceptable excipient. The pharmaceutical composition of the present invention can be used as a medicament, in particular for the treatment and/or prophylaxis of a condition selected from the group consisting of endometriosis, uterine fibroids and prostate cancer.

### BACKGROUND OF THE INVENTION

Relugolix is an oral selective antagonist of the gonadotropin-releasing hormone (GnRH) receptor and currently under development for the treatment of endometriosis, uterine fibrioids and prostate cancer. It can be chemically designated as 1-[4-[1-(2,6-difluorobenzyl)-5-(dimethylaminomethyl)-3-(6-methoxypyridazin-3-yl)-2,4-dioxo-1,2,3,4-tetrahydrothieno[2,3-*d*]pyrimidin-6-yl]phenyl]-3-methoxyurea and is represented by the following chemical structure according to Formula (I)

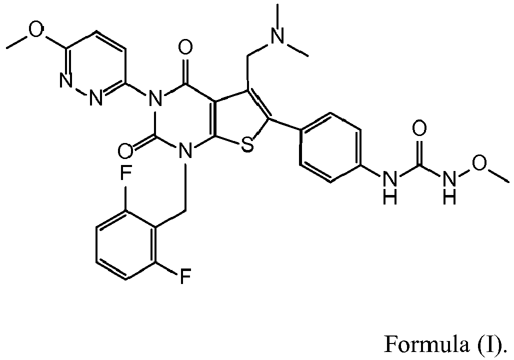

The compound relugolix is disclosed in WO 2004/067535 A1. According to example 83 relugolix was prepared in analogy to the method disclosed in example 4, which involves final recrystallization from dichloromethane/methanol/diethyl ether. Example 83 teaches that relugolix was obtained as colorless crystals.

Miwa et. al, J. Med. Chem. 2011, 54, 4998-5012 describes colorless crystals having a melting point of 228 °C.

WO 2014/051164 A2 discloses a THF solvate and a further crystalline form of relugolix, respectively. While the THF solvate is obtained via crystallization from THF, the latter is produced by recrystallizing the THF solvate from a solvent mixture comprising an alkyl alcohol (e.g. ethanol) and a solvent selected from the group consisting of dimethylsulfoxide, dimethylfromamide and dimethylacetamide (e.g. DMSO).

A compound intended to be used for the preparation of an oral solid dosage form requires particular physical properties, which ensure safe production and storage of the finished dosage form. These physical properties are mainly determined by the crystal structure of the active pharmaceutical ingredient and include, but are not limited to physical stability, hygroscopicity, solubility, dissolution rate, flowability and compactability.

Although solvate or hydrate formation may be used as a means for customizing the physicochemical properties of an active pharmaceutical ingredient with a process or clinical need, they often suffer from certain drawbacks, which to a certain degree compromizes their utility for formulation into a pharmaceutical dosage form. For example, like the THF solvate of WO 2014/051164 A2 they can contain unacceptable amounts of residual organic solvents.

Hence, there remains a need for improved solid-state forms of relugolix possessing physicochemical properties which allow for the stable formulation of relugolix into a pharmaceutical dosage form such that reliable quality and efficacy are achieved throughout shelf-life.

### SUMMARY OF THE INVENTION

The present invention relates to a *hemi*hydrate of relugolix. The relugolix *hemi*hydrate of the present invention possesses one or more favorable physicochemical properties for a drug substance intended for use in an oral solid dosage form selected from the group consisting of chemical stability, physical stability, hygroscopicity, solubility, dissolution, morphology, crystallinity, flowability, compactibility and wettability.

In particular, the relugolix *hemi*hydrate of the present invention is physically and chemically stable and can thus be reliably formulated into a pharmaceutical dosage form and safely stored.

### Abbreviations

- PXRD: powder X-ray diffractogram
- DSC: differential scanning calorimetry
- TGA: thermogravimetric analysis
- GC: gas chromatography
- MS: mass spectrometry
- SXRD: single crystal X-ray diffraction
- NMR: nuclear magnetic resonance
- RT: room temperature
- w-%: weight percent

### Definitions

In the context of the present invention the following definitions have the indicated meaning, unless explicitly stated otherwise:
As used herein the term "room temperature" refers to a temperature in the range of from 20 to 30 °C.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-80% relative humidity, preferably 30-70% relative humidity, more preferably 40-60% relative humidity and most preferably 50% relative humidity.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 9.4° 2-Theta for example can appear between 9.2° and 9.6° 2-Theta, preferably between 9.3 and 9.5° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The crystalline relugolix *hemi*hydrate of the present invention may be referred to herein as being characterized by a powder X-ray diffractogram "as shown in" a figure. The person skilled in the art understands that factors such as variations in instrument type, response and variations in sample directionality, sample concentration, sample purity, sample history and sample preparation may lead to variations, for example relating to the exact reflection and peak positions and their intensities. However, a comparison of the graphical data in the figures herein with the graphical data generated for an unknown physical form and the confirmation that two sets of graphical data relate to the same crystal form is well within the knowledge of a person skilled in the art.

The term "solid-state form" as used herein refers to any crystalline and/or amorphous phase of a compound.

The term "solvate" as used herein, refers to a crystalline solid were either one or more organic solvent(s) is/are cooperated in or accommodated by the crystal structure e.g. is/are part of the crystal structure or entrapped into the crystal (solvent inclusions). Thereby, the one or more organic solvent(s) can be present in a stoichiometric or non-stoichiometric amount. When the one or more organic solvent(s) is/are present in stoichiometric amount(s), the solvate may be referred to by adding greek numeral prefixes. For example, a solvate may be referred to as a *hemi*solvate or as a *mono*solvate depending on the solvent(s)/compound stoichiometry. The solvent content can be measured/determined, for example, by GC, NMR, SXRD and/or TGA/MS.

The term "hydrate" as used herein, refers to a crystalline solid where either water is cooperated in or accommodated by the crystal structure e.g. is part of the crystal structure or entrapped into the crystal (water inclusions). Thereby, water can be present in a stoichiometric or non-stoichiometric amount. When water is present in stoichiometric amount, the hydrate may be referred to by adding greek numeral prefixes. For example, a hydrate may be referred to as a *hemi*hydrate or as a *mono*hydrate depending on the water/compound stoichiometry. The water content can be measured, for example, by Karl-Fischer-Coulometry, GC, NMR, SXRD and/or TGA/MS.

The terms "dehydrating" or "dehydration" as used herein, describe the at least partial removal of water from the crystal structure of the host molecule.

A "predetermined amount" as used herein with regards to the relugolix *hemi*hydrate of the present invention refers to the initial amount of the relugolix *hemi*hydrate used for the preparation of a pharmaceutical composition having a desired dosage strength of relugolix.

The term "effective amount" as used herein with regards to the relugolix *hemi*hydrate of the present invention encompasses an amount of relugolix *hemi*hydrate, which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

### BRIEF DESCRIPTION OF THE FIGURES

**Figure 1****:** illustrates a representative PXRD of the relugolix *hemi*hydrate of the present invention. The x-axis shows the scattering angle in °2-Theta, the y-axis shows the intensity of the scattered X-ray beam in counts of detected photons.
**Figure 2****:** illustrates a representative TGA curve of the relugolix *hemi*hydrate of the present invention. The x-axis shows the temperature in degree Celsius (°C), the y-axis shows the mass (loss) of the sample in weight percent (w-%).
**Figure 3****:** illustrates a representative DSC curve of the relugolix *hemi*hydrate of the present invention. The x-axis shows the temperature in degree Celsius (°C), the y-axis shows the heat flow rate in Milliwatt per gram (W/g) with endothermic peaks going up.

### DETAILED DESCRIPTION OF THE INVENTION

The present invention relates to a hydrate of relugolix. In particular, the present invention relates to a *hemi*hydrate of relugolix herein also designated as "relugolix *hemi*hydrate". More precisely, the present invention relates to relugolix *hemi*hydrate, characterized by the chemical structure according to Formula (II) 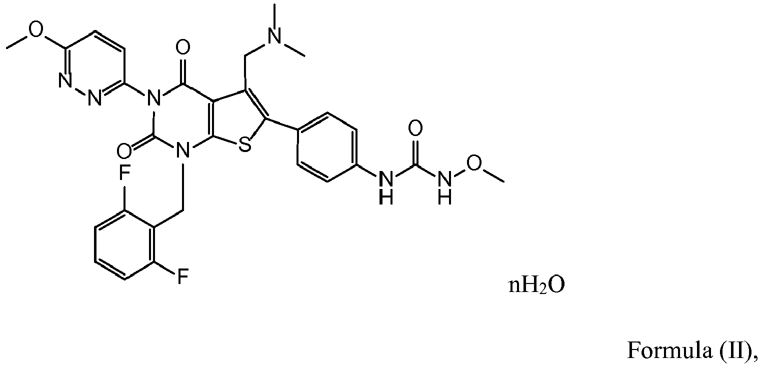 wherein n is in the range of from 0.3 to 0.7, preferably in the range of from 0.4 to 0.6 more preferably in the range of from 0.45 to 0.55 and most preferably n is about 0.5. For example, n can be selected from the group consisting of about 0.3, 0.4, 0.45, 0.5, 0.55, 0.6 and 0.7. The relugolix *hemi*hydrate of the present invention has a molar ratio of relugolix and water in the range of from 1.0: 0.3 to 0.7, preferably of from 1.0: 0.4 to 0.6, more preferably of from 1.0: 0.45 to 0.55 and most preferably the molar ratio is about 1.0 to 0.5.

The relugolix *hemi*hydrate of the present invention as defined in any one of the above described embodiments may be characterized by analytical methods well known in the field of the pharmaceutical industry for characterizing solids. Such methods comprise but are not limited to powder X-ray diffraction, differential scanning calorimetry and thermogravimetric analysis. It may be characterized by one of the aforementioned analytical methods or by combining two or more of them. In particular, the relugolix *hemi*hydrate of the present invention may be characterized by any one of the following embodiments or by combining two or more of the following embodiments.

The present invention relates to relugolix *hemi*hydrate, characterized by having a PXRD comprising reflections at 2-Theta angles of:
(5.9 ± 0.2)°, (7.5 ± 0.2)° and (9.4 ± 0.2)°; or
(5.9 ± 0.2)°, (6.9 ± 0.2)°, (7.5 ± 0.2)° and (9.4 ± 0.2)°; or
(5.9 ± 0.2)°, (6.9 ± 0.2)°, (7.5 ± 0.2)°, (9.4 ± 0.2)° and (12.1 ± 0.2)°; or
(5.9 ± 0.2)°, (6.9 ± 0.2)°, (7.5 ± 0.2)°, (9.4 ± 0.2)°, (12.1 ± 0.2)° and (16.5 ± 0.2)°; or
(5.9 ± 0.2)°, (6.9 ± 0.2)°, (7.5 ± 0.2)°, (9.4 ± 0.2)°, (12.1 ± 0.2)°, (16.5 ± 0.2)° and (18.1 ± 0.2)°; or
(5.9 ± 0.2)°, (6.9 ± 0.2)°, (7.5 ± 0.2)°, (9.4 ± 0.2)°, (12.1 ± 0.2)°, (16.5 ± 0.2)°, (18.1 ± 0.2)° and (19.1 ± 0.2)°; or
(5.9 ± 0.2)°, (6.9 ± 0.2)°, (7.5 ± 0.2)°, (9.4 ± 0.2)°, (10.2 ± 0.2)°, (12.1 ± 0.2)°, (16.5 ± 0.2)°, (18.1 ± 0.2)° and (19.1 ± 0.2)°; or
(5.9 ± 0.2)°, (6.4 ± 0.2)°, (6.9 ± 0.2)°, (7.5 ± 0.2)°, (9.4 ± 0.2)°, (10.2 ± 0.2)°, (12.1 ± 0.2)°, (16.5 ± 0.2)°, (18.1 ± 0.2)° and (19.1 ± 0.2)°;
when measured at a temperature in the range of from 20 to 30 °C with Cu-Kalpha_{1,2} radiation having a wavelength of 0.15419 nm.

Alternatively, the present invention relates to relugolix *hemi*hydrate, characterized by having a PXRD comprising reflections at 2-Theta angles of:
(5.9 ± 0.1)°, (7.5 ± 0.1)° and (9.4 ± 0.1)°; or
(5.9 ± 0.1)°, (6.9 ± 0.1)°, (7.5 ± 0.1)° and (9.4 ± 0.1)°; or
(5.9 ± 0.1)°, (6.9 ± 0.1)°, (7.5 ± 0.1)°, (9.4 ± 0.1)° and (12.1 ± 0.1)°; or
(5.9 ± 0.1)°, (6.9 ± 0.1)°, (7.5 ± 0.1)°, (9.4 ± 0.1)°, (12.1 ± 0.1)° and (16.5 ± 0.1)°; or
(5.9 ± 0.1)°, (6.9 ± 0.1)°, (7.5 ± 0.1)°, (9.4 ± 0.1)°, (12.1 ± 0.1)°, (16.5 ± 0.1)° and (18.1 ± 0.1)°; or
(5.9 ± 0.1)°, (6.9 ± 0.1)°, (7.5 ± 0.1)°, (9.4 ± 0.1)°, (12.1 ± 0.1)°, (16.5 ± 0.1)°, (18.1 ± 0.1)° and (19.1 ± 0.1)°; or
(5.9 ± 0.1)°, (6.9 ± 0.1)°, (7.5 ± 0.1)°, (9.4 ± 0.1)°, (10.2 ± 0.1)°, (12.1 ± 0.1)°, (16.5 ± 0.1)°, (18.1 ± 0.1)° and (19.1 ± 0.1)°; or
(5.9 ± 0.1)°, (6.4 ± 0.1)°, (6.9 ± 0.1)°, (7.5 ± 0.1)°, (9.4 ± 0.1)°, (10.2 ± 0.1)°, (12.1 ± 0.1)°, (16.5 ± 0.1)°, (18.1 ± 0.1)° and (19.1 ± 0.1)°;
when measured at a temperature in the range of from 20 to 30 °C with Cu-Kalpha_{1,2} radiation having a wavelength of 0.15419 nm.

In addition, the present invention relates to relugolix *hemi*hydrate, characterized by having a PXRD comprising reflections at 2-Theta angles of (6.9 ± 0.2)°, (7.3 ± 0.2)°, (7.5 ± 0.2)°, (9.4 ± 0.2)°, (9.7 ± 0.2)°, (12.1 ± 0.2)°, (16.5 ± 0.2)° (18.1 ± 0.2)°, (19.1 ± 0.2)° and (23.2 ± 0.2)°, when measured at a temperature in the range of from 20 to 30 °C with Cu-Kalpha_{1,2} radiation having a wavelength of 0.15419 nm.

Alternatively, the present invention relates to relugolix *hemi*hydrate, characterized by having a PXRD comprising reflections at 2-Theta angles of (6.9 ± 0.1)°, (7.3 ± 0.1)°, (7.5 ± 0.1)°, (9.4 ± 0.1)°, (9.7 ± 0.2)°, (12.1 ± 0.1)°, (16.5 ± 0.1)° (18.1 ± 0.1)°, (19.1 ± 0.1)° and (23.2 ± 0.1)°, when measured at a temperature in the range of from 20 to 30 °C with Cu-Kalpha_{1,2} radiation having a wavelength of 0.15419 nm.

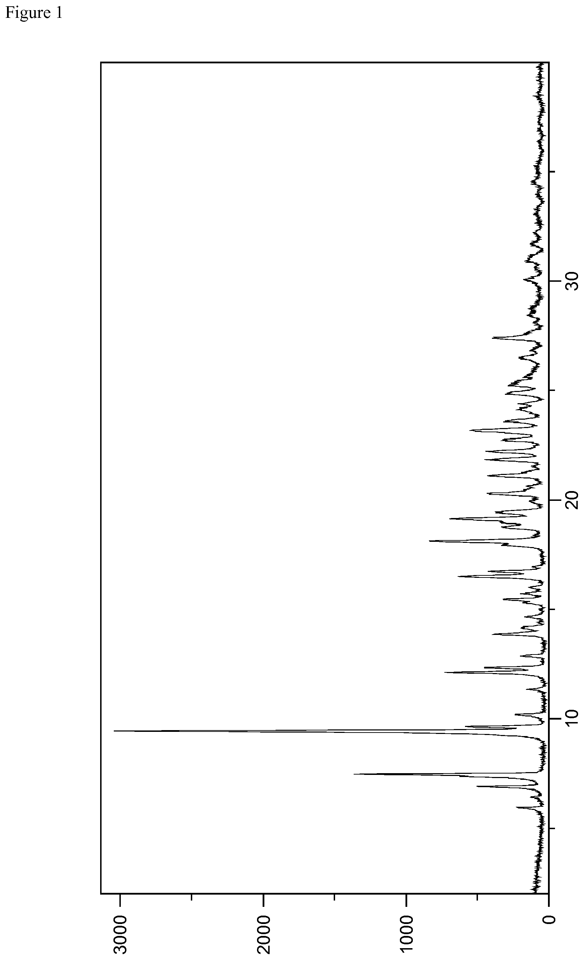

The present invention also relates to relugolix *hemi*hydrate, characterized by having a PXRD essentially the same as shown in Figure 1 of the present invention, when measured at a temperature in the range of from 20 to 30 °C with Cu-Kalpha_{1,2} radiation having a wavelength of 0.15419 nm.

Furthermore, the present invention also relates to relugolix *hemi*hydrate, characterized by having a DSC curve comprising an endothermic peak, preferably a first endothermic peak, having an onset at a temperature of (67 ± 5)°C, preferably of (67 ± 3)°C, even more preferably of (67 ± 2)°C and most preferably of (67 ± 1)°C, when measured a heating rate of 10 K/min.

The present invention also relates to relugolix *hemi*hydrate, characterized by having a DSC curve comprising an endothermic peak, preferably a first endothermic peak, having a peak maximum at a temperature of (96 ± 5)°C, preferably of (96 ± 3)°C, even more preferably of (96 ± 2)°C and most preferably of (96 ± 1)°C, when measured a heating rate of 10 K/min.

Moreover, the invention relates to relugolix *hemi*hydrate, characterized by having a DSC curve comprising an endothermic peak, preferably a first endothermic peak, with an enthalpy of (36 ± 5) J/g, preferably of (36 ± 3) J/g, even more preferably of (36 ± 2) J/g and most preferably of (36 ± 1) J/g, when measured at a heating rate of 10 K/min.

In another embodiment, the present invention relates to relugolix *hemi*hydrate, characterized by having a TGA curve showing a mass loss in the range of from 0.9 - 2.1 w-%, preferably of from 1.2 - 1.8 w-%, more preferably of from 1.35 - 1.65 w-% and most preferably the mass loss is about 1.5 w-%, based on the weight of the relugolix *hemi*hydrate, when heated from 25 to 120 °C at a rate of 10 K/min.

In a further aspect the present invention relates to the use of the relugolix *hemi*hydrate of the present invention as defined in any one of the embodiments described above for the preparation of a pharmaceutical composition.

In another aspect, the present invention relates to a pharmaceutical composition comprising the relugolix *hemi*hydrate of the present invention as defined in any one of the embodiments described above, preferably in an effective and/or predetermined amount, and at least one pharmaceutically acceptable excipient. Preferably, the pharmaceutical composition of the present invention is an oral solid dosage form, such as a tablet or a capsule. More preferably, the pharmaceutical composition of the present invention is a tablet e.g. a film-coated tablet.

The at least one pharmaceutically acceptable excipient, which is comprised in the pharmaceutical composition of the present invention is preferably selected from the group consisting of fillers/diluents, binders, disintegrants, lubricants, stabilizers, coating materials and combinations thereof. Preferably, all of these pharmaceutically acceptable excipients are comprised by the pharmaceutical composition of the present invention.

In a preferred embodiment, the at least one pharmaceutically acceptable excipient, which is comprised in the pharmaceutical composition of the present invention is selected from the group consisting of *D*-mannitol, crystalline cellulose, hydroxypropyl cellulose, crosscarmellose sodium, magnesium stearate and one or more coating materials. More preferably, all of these pharmaceutically acceptable excipients are comprised by the pharmaceutical composition of the present invention. Preferably, the coating materials comprise hydroxypropylmethyl cellulose, titanium dioxide and ferric oxide.

In one embodiment, the present invention relates to a pharmaceutical composition as defined in any one of the embodiments described above, wherein the pharmaceutical composition comprises not less than 25 w-%, preferably not less than 35 w-%, more preferably not less than 40 w-% of relugolix *hemi*hydrate calculated as water-free relugolix, based on the weight of the pharmaceutical composition. Preferably, the pharmaceutical composition comprises not more than 80 w-%, more preferably not more than 75 w-% of relugolix *hemi*hydrate calculated as water-free relugolix, based on the weight of the pharmaceutical composition.

Furthermore, the present invention relates to a pharmaceutical composition as describe above, wherein the predetermined and/or effective amount of relugolix *hemi*hydrate is selected from the group consisting of 20 mg, 30 mg, 40 mg, 50 mg, 60 mg, 70 mg, 80 mg, 90 mg, 100 mg, 110 mg, 120 mg, 130 mg, 140 mg, 150 mg and 160 mg calculated as water-free relugolix. Preferably, the invention relates to a pharmaceutical composition as describe above, wherein the predetermined and/or effective amount of relugolix *hemi*hydrate is selected from the group consisting of 40 mg, 50 mg, 60 mg, 70 mg, 80 mg, 90 mg, 100 mg, 110 mg and 120 mg, calculated as water-free relugolix. Most preferably, the predetermined and/or effective amount of relugolix *hemi*hydrate is 40 mg, 80 mg or 120 mg, calculated as water-free relugolix.

The present invention also relates to a pharmaceutical composition as defined in any one of the above described embodiments, wherein the pharmaceutical composition is to be administered once-daily.

In a further aspect, the present invention relates to the pharmaceutical composition as defined in any one of the above described embodiments for use as a medicament.

In yet another aspect, the present invention relates to the pharmaceutical composition as defined in any one of the above described embodiments for use in the treatment or prophylaxis of a condition selected from the group consisting of endometriosis, uterine fibroids and prostate cancer.

In another embodiment, the present invention is directed to a method of treating or prophylactically preventing a condition selected from the group consisting of endometriosis, uterine fibroids and prostate cancer by administering the pharmaceutical composition as defined in any one of the above described embodiments to a patient in need of such a treatment and/or prophylaxis.

### EXAMPLES

The following non-limiting examples are illustrative for the disclosure and are not to be construed as to be in any way limiting for the scope of the invention.

### Analytical Methods

Powder X-ray diffraction was performed with a PANalytical X' Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

TGA was performed on a Mettler TGA/DSC 1 instrument. Relugolix *hemi*hydrate (11.4 mg) was heated in a 100 microliter aluminum pan closed with an aluminum lid. The lid was automatically pierced at the beginning of the measurement. The sample was initially kept at 25 °C for 2.5 minutes and then heated from 25 to 150 °C at a rate of 10 K/min. Nitrogen (purge rate 30 mL/min) was used as purge gas.

DSC was performed on a Mettler Polymer DSC R instrument. Relugolix *hemi*hydrate (3.3 mg) was heated in a 40 microliter aluminium pan with a pierced aluminium lid from 25 to 250 °C at a rate of 10 K/min. Nitrogen (purge rate 50 mL/min) was used as purge gas.

### Example 1: Preparation of 1-[4-[1-(2,6-difluorobenzyl)-5-(dimethylaminomethyl)-3-(6-methoxypyridazin-3-yl)-2,4-dioxo-1,2,3,4-tetrahydrothieno[2,3-u0pyrimidin-6-yl]phenyl]-3-methoxyurea hemihydrate (relugolix hemihydrate)

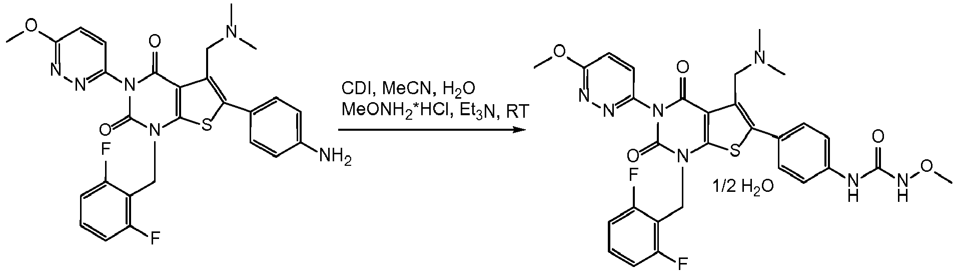

1,1'-Carbonyldiimidazole (CDI) (16.27 g, 0.1003 mol) was dissolved in acetonitrile (130 mL) and stirred well. Triethylamine (Et3N) (7.4 mL, 0.0531 mol, 0.9 equiv) was added with stirring and then the reaction mixture was cooled to 10 °C. Methoxyamine hydrochloride (MeONH₂*HCl) (9.414 g, 0.1127 mol) was added portion-wise by not exceeding 30 °C inner temperature. The reaction mixture was stirred well until complete dissolution. 6-(4-Aminophenyl)-1-(2,6-difluorobenzyl)-5-(dimethylaminomethyl)-3-(6-methoxypyridazin-3-yl)thieno[2,3-*d*]pyrimidin-2,4(1*H*, 3*H*)-dione (32.49 g, 0.0590 mol, e.g. prepared according to Examples 6 or 14 of WO 2014/051164 A2) was then added to the reaction mixture. The residual material in the flask was then taken up in acetonitrile (20 mL) and the washings transferred to the reaction mixture. This reaction mixture was warmed to 50 °C (internal temperature) and stirred for 16 hours.

Work-up: Triethylamine (Et3N) (10.69 mL, 0.0767 mol, 1.3 equiv.) was added at 50 °C followed by water (150 mL) portionwise and the mixture was stirred for 3 hours at 50 °C. Thereafter, further amount of water (300 mL) was added at the same temperature and then it was allowed to stir at RT overnight. The solid was filtered under vacuum, washed with water (2 x 150 mL) and dried in vacuum. Solid-state characterization of the obtained material is described in Example 2 hereinafter.

### Example 2: Solid-state characterization of relugolix hemihydrate obtained according to Example 1

### Powder X-ray diffraction

A representative diffractogram of relugolix *hemi*hydrate is displayed in Figure 1 herein. The corresponding reflection list is provided in Table 1 below.

**Table 1: PXRD reflections of relugolix hemihydrate in the range of from 2 to 30° 2-Theta; A typical precision of the 2-Theta values is in the range of ± 0.2° 2-Theta, preferably of ± 0.1° 2-Theta.**

| **Reflection position [°2-Theta]** | **Reflection position [°2-Theta]** | **Reflection position [°2-Theta]** | **Reflection position [°2-Theta]** |
|---|---|---|---|
| 5.9 | 12.9 | 18.1 | 23.2 |
| 6.4 | 13.9 | 18.8 | 23.6 |
| 6.9 | 14.2 | 19.1 | 24.1 |
| 7.3 | 14.7 | 19.5 | 24.4 |
| 7.5 | 15.3 | 19.9 | 24.9 |
| 9.4 | 15.5 | 20.3 | 25.2 |
| 9.7 | 15.7 | 20.6 | 25.4 |
| 10.2 | 16.0 | 21.1 | 26.5 |
| 11.3 | 16.5 | 21.9 | 27.4 |
| 12.1 | 16.7 | 22.2 | 28.5 |
| 12.3 | 17.9 | 22.7 | 28.7 |

### Thermoanalysis

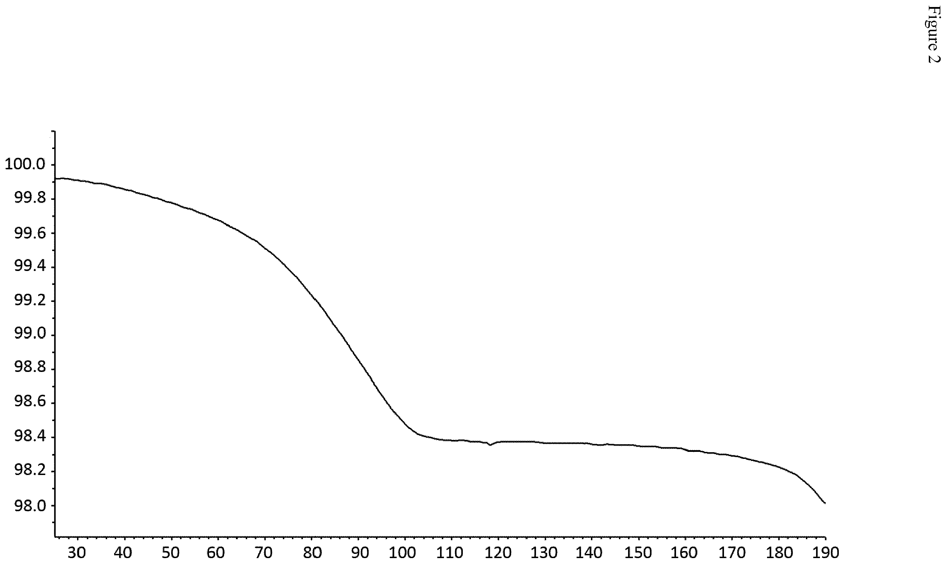

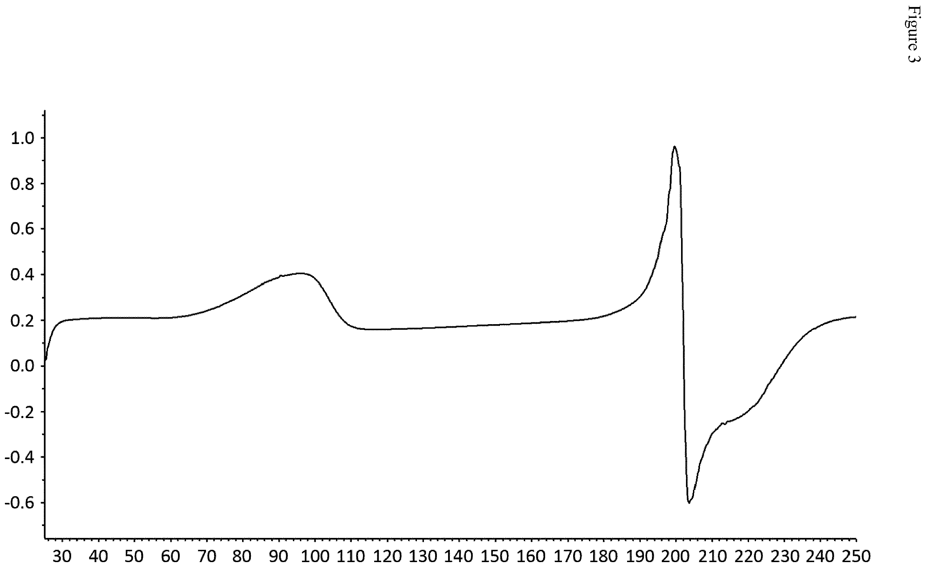

The TGA curve of relugolix *hemi*hydrate is displayed in Figure 2 of the present invention and shows a mass loss of about 1.5 w-% from the start of the measurement until about 120 °C, which corresponds to about 0.5 mol equivalent of water. The weight loss corresponds well to the broad endothermic peak observed in the DSC curve displayed in Figure 3 herein (onset: 67.1 °C, peak:96.2 °C, DH: 36.2 J/g), which can be assigned to the dehydration process. Thermoanalysis indicates that relugolix *hemi*hydrate of the present invention is physically stable upon moderate temperature stress.

### Example 3: Film-coated tablets comprising relugolix hemihydrate

In a fluid bed granulator relugolix *hemi*hydrate, *D*-mannitol and crystalline cellulose were mixed. An aqueous solution of hydroxypropyl cellulose was sprayed onto the blend and the mixture was dried to give a granulated powder. The granulated powder was milled to give a milled powder. Croscarmellose sodium and magnesium stearate were added to the obtained milled powder and they were mixed to give a mixed powder. The mixed powder was tableted by a rotary tableting machine to give core tablets. The core tablets were film-coated to yield film-coated tablets.

| **Ingredient** | **mg/tablet** |
|---|---|
| Relugolix *hemi*hydrate | 81.2* |
| *D*-mannitol | 125 |
| crystalline cellulose | 51 |
| hydroxypropyl cellulose | 7.6 |
| croscarmellose sodium | 12.7 |
| magnesium stearate | 2.5 |
| **Tablet core** | **280** |
| hydroxypropylmethyl cellulose | 9.06 |
| titanium oxide | 1.02 |
| red ferric oxide | 0.10 |
| **Film-coating** | **10.2** |
| **Film Coated Tablet** | **290.2** |

| | |
|---|---|
| *Equivalent to 80 mg relugolix | |

## Claims

1. Relugolix *hemi*hydrate.

2. The relugolix *hemi*hydrate of claim 1, **characterized by** the chemical structure according to Formula (II) 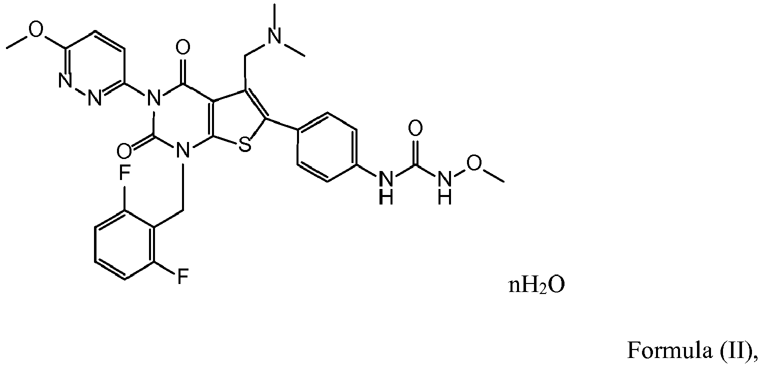 wherein n is in the range of from 0.3 to 0.7.

3. The relugolix *hemi*hydrate of claim 1 or 2, **characterized by** having a powder X-ray diffractogram comprising reflections at 2-Theta angles of (5.9 ± 0.2)°, (7.5 ± 0.2)° and (9.4 ± 0.2)°, when measured at a temperature in the range of from 20 to 30 °C with Cu-Kalpha_{1,2} radiation having a wavelength of 0.15419 nm.

4. The relugolix *hemi*hydrate of claim 3 **characterized by** having a powder X-ray diffractogram comprising additional reflections at 2-Theta angles of (6.9 ± 0.2)° and (12.1 ± 0.2)°, when measured at a temperature in the range of from 20 to 30 °C with Cu-Kalpha_{1,2} radiation having a wavelength of 0.15419 nm.

5. The relugolix *hemi*hydrate as defined in any one of the preceding claims, **characterized by** having a differential scanning calorimetry curve comprising a first endothermic peak having an onset at a temperature of (67 ± 5)°C, when measured at a heating rate of 10 K/min.

6. The relugolix *hemi*hydrate as defined in any one of the preceding claims, **characterized by** having a thermogravimetric analysis curve showing a mass loss in the range of from 0.9 - 2.1 w-%, based on the weight of the relugolix *hemi*hydrate, when heated from 25 to 120 °C at a rate of 10 K/min.

7. Use of the relugolix *hemi*hydrate as defined in any one of the preceding claims for the preparation of a pharmaceutical composition.

8. A pharmaceutical composition comprising the relugolix *hemi*hydrate as defined in any one of claims 1 to 6 and at least one pharmaceutically acceptable excipient.

9. The pharmaceutical composition as defined in claim 8, wherein the pharmaceutical composition is an oral solid dosage form.

10. The pharmaceutical composition of claim 9, wherein the oral solid dosage form is a film-coated tablet.

11. The pharmaceutical composition as defined in any one of claims 8 to 10, comprising 40 mg, 80 mg or 120 mg of the relugolix *hemi*hydrate as defined in any one of claims 1 to 6, calculated as water-free relugolix.

12. The pharmaceutical composition as defined in any one of claims 8 to 11 for use as a medicament.

13. The pharmaceutical composition as defined in any one of claims 8 to 11 for use in the treatment of a condition selected from the group consisting of endometriosis, uterine fibroids and prostate cancer.
